# EUROPEAN PATENT SPECIFICATION

(11) **EP 1 656 888 B1**
(45) Date of publication and mention of the grant of the patent: **13.01.2010**
(21) Application number: 05024769.1
(22) Date of filing: 12.11.2005
(51) Int. Cl.: A61B 10/00, A61B 17/32

(54) **Device for cutting out tissue specimens**
Biopsievorrichtung
Instrument de biopsie

(30) Priority: 15.11.2004 EP 04027137; 16.09.2005 EP 05020235
(43) Date of publication of application: 17.05.2006
(73) Proprietor: Roche Diagnostics GmbH, 68305 Mannheim (DE); F. Hoffmann-La Roche AG, 4070 Basel (CH)
(72) Inventor: Eberle, Walter, 82347 Bernried (DE); Hloch, Peter, 82431 Kochel (DE); Hundt, Christoph, 81371 München (DE); Rainer, Alois, 80939 München (DE)
(74) Representative: Knauer, Martin

(56) References cited:
- EP-A- 1 293 167
- US-A1- 2003 082 797
- US-A1- 2004 116 942

## Description

The present invention relates to the use of a cutting device with which a quantitatively defined tissue specimen can be removed from a larger tissue specimen or tissue mass, from brain tissue obtained post mortem from resected brainstem. A preferred embodiment is a method for separating a tissue part involving a cutting device for one-off use. The invention further concerns a method for removing quantitatively defined tissue specimens from a larger tissue specimen or tissue mass using the cutting device.

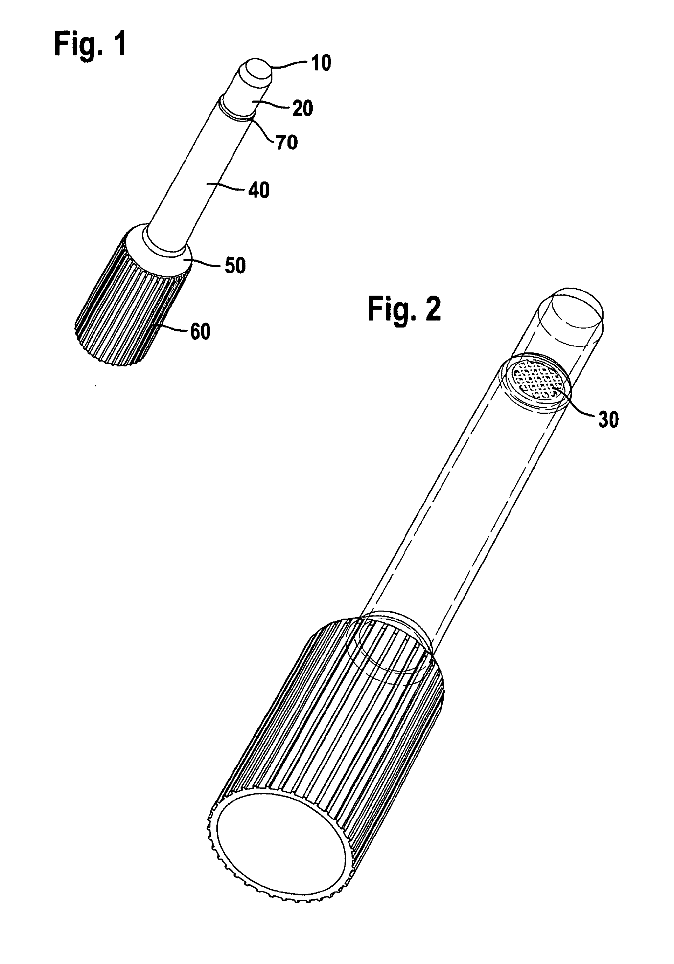

After slaughter, cows over a certain age are presently examined for BSE (prion infection) with the aid of an immunological test, e.g. the Prionics^{®}-Check LIA, Prionics^{®}-Check WESTERN or PrionScreen BSE assays (PrionScreen is a Trademark of Roche). The cadaver is released for further processing and allowed to enter the human food chain upon obtaining a negative test result. Part of the brainstem (medulla oblongata), preferably the obex region (Figure 1), is used as starting material for removal of a tissue specimen with which the test is carried out. Part of the brainstem is removed in the slaughterhouse. By way of the foramen magnum, i.e. the large opening on the posterior of the skull through which the spinal cord emerges, the obex and adjacent areas are removed using a specially shaped spoon and are introduced into a special container. In the container, the brain material is taken to a test laboratory where the actual specimen for establishing a prion infection is removed.

A number of test systems presently available on the market require the analysis to be carried out with a defined quantity of specimen material. The quantity is generally determined by weighing. To do this, manual operations have to be conducted in which potentially infectious material is handled under stringent biological safety conditions. Particularly with a view to safety at work, it is desirable to limit the operations using unsealed brain material. In concrete terms, it would be advantageous to be able to determine the quantity more quickly and more easily since, in this way, more specimens can be fed to a test system per unit of time. This would have a positive impact on the specimen throughput attainable in the laboratory.

In the state of the art there are devices known which were designed for taking samples from tissue or a tissue specimen. However, the state of the art has certain disadvantages.

EP 1 293 167 discloses a cutting device for biopsies comprising a hollow needle with a circular blade. A grid inside the needle limits the amount of tissue which can be taken up by the cavity of the needle. Also inside the needle, between the grid and the opening of the needle a thin wire is stretched between two opposite points at the inner wall of the needle. After the device is pushed into a tissue specimen the needle is twisted, whereby the tissue portion located between the grid and the wire is cut from the remaining tissue specimen. Suction is applied to retain the cut portion of tissue at the grid, thereby allowing to remove the cut portion from the remaining tissue specimen.

US 2004/0116942 discloses a device for extraction of follicular units from a donor area, for the purpose of transplantation into balding areas of the scalp. The device comprises a cylindrical punch to which a perforator is affixed, and a grid as a guard member aimed at containing successfully extracted hair follicles. The punch is described as being a standard biopsy punch with a diameter of 1 mm, corresponding to the size of a single target follicular unit. The punch is used to make a first circular incision around the hair follicle. The perforator is used to make a second, deeper incision to weaken the structural integrity of the surrounding connective tissue without damaging the hair follicle. Subsequently, the follicular unit is extracted using suction. In the device the grid prevents the follicular unit to enter the suction means and be eventually lost.

Another device for removing tissue parts is disclosed in US 3,990,451 and US 2003/082797.

US 2003/082797 discloses a sampling device for sampling soft biological material such as brain material post mortem. A hollow cylindrical device is equipped with a slicing section at one end. The slicing section comprises (i) a slicing edge and (ii) cutting wires arranged diametrically in the plane of the end of the cylinder. Within the cylinder there is a movable piston which is controlled by a rod. The piston rod emerges from the opposite end of the cylinder. When applied to soft tissue material, the cylinder is pressed into the tissue material and there is a dual cutting process. On the one hand, the slicing edge of the cylinder divides a cylindrical tissue core from the surrounding tissue material; on the other hand, the cutting wire or wires slice through the cylindrical core. While the cylinder is pressed into the tissue material, the tissue core displaces the piston towards the end of the cylinder opposite to the slicing section. Before the cylinder is pulled out, it is subjected to a rotating movement. This movement ensures that the body of the tissue core inside the cylinder is disconnected from the remaining tissue mass. Thus, the tissue core remains inside the cylinder when it is pulled out of the material. By way of actuating the rod and moving the piston towards the slicing section the tissue core can be released from the cylinder.

It was an object of the present invention to make available a simple device which can be produced as a disposable and with which a quantitatively defined tissue specimen can be obtained from potentially infectious brain material quickly and reliably and with reduced manual work. A further object of the invention was to particularly minimize the risk of injury during the sampling process as well as the risk of contamination. A further object was to make available a method in which the number of manual steps leading to a quantitatively defined tissue specimen is reduced. A further object of the invention was to make available a method in which the steps leading to a quantitatively defined tissue specimen are simplified and speeded up.

According to the invention, the problem was solved by provision of a method according to claim 1.

### Detailed description

In the method of the invention, the cutting device comprises a circular blade which continues as the wall of a cylindrical or substantially cylindrical hollow body, at the end of which a grid is arranged perpendicular to the cylinder axis. The cutting device also comprises a shaft which continues the cylinder axis on the other side of the grid in the direction of the longitudinal axis (extending from the cutting edge to the grid). The shaft can be used as a handle when using the device. In a preferred embodiment, the shaft is a hollow body which is open at both ends. A particularly preferred embodiment of the method according to the invention makes use of a device for separating a tissue part from brain material shown in Figure 1 and Figure 2, **characterized in that** said device is a hollow, tube-shaped entity comprising (i) at the front end a circular blade (10) which continues as the wall of a cylindrical or substantially cylindrical body (20), at the end of which a grid (30) is arranged perpendicular to the cylinder axis; (ii) a cylindrical or substantially cylindrical shaft (40) which is joined with the cylindrical body (20) on the other side of the grid (30); (iii) at the rear end a cylindrical or substantially cylindrical handle (60) which is joined with the shaft by a widening part (50) with the surface of a truncated cone whose ends are circular, the end of the smaller circle diameter starting flush on the rear end of the shaft, and the end with the larger circle diameter starting flush on the handle. The device in the method according to the invention is combined with (iv) a smooth fixed support against which the circular blade can be pressed.

In a preferred embodiment, the grid (30) contains at least 5 elements with openings. It is preferred to have 15 to 30 elements with openings. It is also preferred that the width of each grid bar is between 0.2 mm and 1 mm, very much preferred 0.4 mm.

In a preferred embodiment, the shaft comprises a cylindrical or substantially cylindrical hollow body which starts on the cylinder of the blade on the other side of the grid. In a further preferred embodiment, the shaft has a further widening part (70) with the surface of a truncated cone whose ends are circular, the end with the smaller circle diameter starting flush on the cylinder of the blade on the other side of the grid, and the end with the larger diameter starting flush on a cylindrical or substantially cylindrical hollow body.

In a further preferred embodiment, the shaft comprises (a) a first cylindrical hollow body which starts flush on the cylinder of the blade on the other side of the grid and continues this cylinder in the direction of the longitudinal axis, (b) a widening part with the surface of a truncated cone whose ends are circular, the end with the smaller circle diameter starting on the cylinder of (a) on the other side of the grid, and the end with the larger diameter starting flush on (c) a cylindrical or substantially cylindrical hollow body.

In a further preferred embodiment the outside diameter of the shaft is at least equal to the outside diameter of the cylindrical body (20). Also preferred, the outside diameter of the shaft is up to 1.5 times wider than the outside diameter of the cylindrical body (20).

In a preferred embodiment the front part of the shaft (40) is joined with the cylindrical body (20) on the other side of the grid (30) by a further widening part (70) with the surface of a truncated cone whose ends are circular, the end of the smaller circle diameter starting flush on the cylindrical body (20) on the other side of the grid (30), and the end with the larger circle diameter starting flush on the front part of the shaft (40). The grid can be adjoined to or be the internal structure of the further widening part (70). In a preferred embodiment said further widening part measures between 1 mm and 5 mm in length and has a widening angle of between 5° and 45°, relative to the wall of the cylindrical body (20). Particular preverence is given to a widening angle of approximately 30°. In this regard, an angle of 1° is understood as 1/360 of the circle circumference.

The handle of the device provides grip for manual operation thus allowing quick, precise and safe handling. In a preferred embodiment the outside diameter of the handle (60) at the rear end is between 1.5 to 10 times wider than the outside diameter of the cylindrical body (20). More preferred, the outside diameter of the handle (60) at the rear end is between 15 mm and 60 mm, even more preferred between 15 mm and 20 mm, even more preferred about 17 mm.

Also preferred, the outside surface of the handle (60) is corrugated longitudinally.

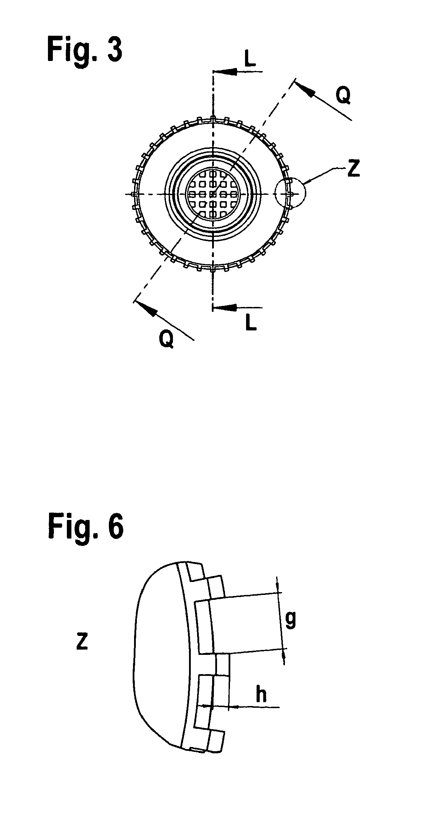

Figure 1, Figure 2, Figure 3, Figure 4, Figure 5 and Figure 6 show details of embodiments of the cutting device for use in the method according to the invention.

Since the cutting device is provided for working with potentially infectious brain material, it is preferably intended to be used only once by the user (i.e. is designed as a disposable item). Disposable items can be advantageously produced in large numbers by injection moulding. Thus, there is disclosed a mould or a plurality of moulds for injection moulding of a device for use in a method according to the invention. Further disclosed is the use of said mould for manufacturing a device according to the invention.

When practicing injection moulding it is preferred to shape hollow tube-like structures not exactly cylindrical but substantially cylindrical. In some preferred cases, the interior of the cutting part of the device is substantially cylindrical. This is to be understood as meaning that the interior tapers from the cutting edge towards the grid. This arrangement is advantageous if the cutting device is made of a plastic material by means of injection moulding. The angle of taper is typically between 0.5° and 10°, preferably between 0.5° and 5°, particularly preferably 1°.

Preferably, the inner wall of the cylindrical body (20) forms the surface of a truncated cone whose ends are circular, the end of the smaller circle diameter facing the grid (30), and the end with the larger circle diameter being the circle formed by the blade, whereby the diameters of the smaller circle and the larger circle are selected such that they result in a truncated cone with an opening angle of between 0.1° and 5°.

It is further preferred that the inside wall of the handle forms the surface of a truncated cone whose ends are circular, the end of the smaller circle diameter facing the widening part (50), whereby the diameters of the smaller circle and the larger circle are selected such that they result in a truncated cone with an opening angle of between 0.1° and 5°. It is further preferred that the inside wall of the shaft forms the surface of a truncated cone whose ends are circular, the end of the smaller circle diameter facing the grid (30) or the further widening part (70), whereby the diameters of the smaller circle and the larger circle are selected such that they result in a truncated cone with an opening angle of between 0.1° and 5°.

Various starting materials used in injection moulding are well known to the skilled person. Because of the required blade function of the cutting edge, hard plastics are more preferable to soft plastics. A plastic material from the group of polycarbonates is preferred for this purpose, particularly preferably the plastic called Makrolon^{®}. The most preferred material is Makrolon^{®} 2458.

As will be described below, the cutting device is used to cut out a tissue part as cylindrical core from a larger tissue mass. The preferred distance between blade edge and grid corresponds substantially to the thickness of the material that is to be cut through. The preferred diameter of the circle formed by the blade and thus of the diameter of the interior will depend on the required quantity of specimen.

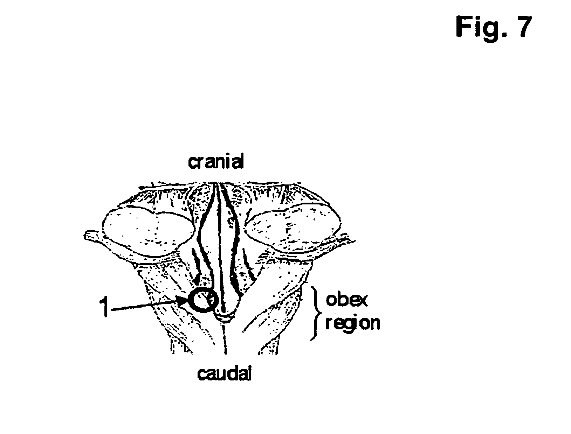

The method of the invention using the cutting tool involves cutting out a cylindrical or substantially cylindrical tissue core from the area of the brainstem referred to as the obex, said tissue core being cut from the obex in the dorsoventral direction at the site (removal site) indicated by reference number (1) in Figure 7, as will be described below.

The brainstem sections from which tissue cores are to be removed as specimens usually come from cattle which are about two years old. But the sizes of the obex region of animals above 2 years of age differ only slightly. Consequently, it is sufficient for a given purpose, for example for taking specimens from brainstem sections of cows which are between 2 and 8 years old, to make the cutting device available in a single defined size (related to the tissue core that is to be produced with it).

The volume of the interior of the cylindrical body (20) from the blade edge to the grid is usually larger than the volume of the tissue core being cut out with the cutting device. Nevertheless, the volume of the interior is preferably adapted to the quantity of brain material needed for the purposes of the test.

For removal of tissue cores, the distance between blade edge and grid is therefore preferably between 5 mm and 20 mm, preferably between 10 mm and 15 mm, particularly preferably about 12 mm. The diameter of the circle formed by the blade is between 3 mm and 10 mm, particularly preferably between 5 mm and 7 mm, particularly preferably 5.8 mm.

The preferred wall thickness of the blade wall and of the grid elements is preferably between 0.3 mm and 2 mm, particularly preferably between 0.5 mm and 1 mm, still more preferably between 0.6 mm and 0.8 mm. The edge of the circular blade is sharp, the cutting edge tapering from the outside inwards at an angle of between 10° and 30°. A preferred angle of taper is between 15° and 25°, particular preference being given to an angle of taper of about 20°.

The wall thickness of the handle is of importance only to the extent that it has to ensure that the cutting device can be handled without breaking. A preferred wall thickness in the handle area is between 0.5 mm and 4 mm.

According to the invention the device is used for separating a tissue part from brain material. To remove a cylindrical or substantially cylindrical tissue core of defined quantity, in other words a quantitatively defined specimen, the section of brainstem is placed with its ventral face on a horizontal, fixed support so that the dorsal face is directed upwards, corresponding to the view of the dorsal face of the medulla oblongata shown in Figure 1. Further information on the site of removal can be found at http://niah.naro.affrc.go.jp/disease/bse/retest_prionics.html. At the site indicated by (1), the circular blade of the cutting device is pressed vertically into the brain material while executing rotation movements until the blade has made complete contact with the support. In this way, a cylindrical or substantially cylindrical tissue mass (tissue core) is separated from the obex region. This procedure is usually done manually.

The grid arranged in the cutting device on the one hand limits the tissue material taken up and on the other hand allows air to escape from the interior of the space formed by the circular blade.

With the aid of tweezers, the section of brainstem is now pushed in the direction of the widening part (50) and in this way, in some circumstances, remaining tough connections of tissue parts to the tissue core are severed. The cutting device is withdrawn from the brainstem section and the tissue core is removed with the aid of tweezers from the interior and transferred to a test container.

At this step, the grid arranged in the punching tool has a further function. When the tissue core is being removed, it avoids the development of an underpressure which would hold back the soft tissue and lead to its tearing. In this way, soft or fragile tissue specimens can also be removed without increased risk of contamination. In addition, while the grid does not limit the amount of tissue being cut out from the brainstem, it prevents the tissue core from moving further inwards when manipulated with forceps.

After use, the cutting device is disinfected and disposed of. For this purpose, the cutting device is preferably autoclaved. The small size of the cutting device in the method of the invention means that the amount of waste material to be disposed of is small. Another or else subsequent preferred way of disposal involves incineration of the cutting device.

The following examples, publications and figures further explain the invention, and the patent claims define the scope of its protection. The described methods are to be understood as examples which even after modifications describe the subject of the invention.

### Description of the figures

- **Figure 1**: Device for performing the method of the invention, exterior view. Reference number (10) circular blade; (20) cylindrical or substantially cylindrical body; (40) shaft; (50) widening part; (60) handle; (70) further widening part connecting (20) and (40).
- **Figure 2**: Preferred cutting device, interior features. Reference number (30) grid.
- **Figure 3**: View along the central longitudinal axis towards the grid; the view is taken through the handle of the device shown in Figure 1 and Figure 2, the point of the observer lying on the central longitudinal axis. L - L and Q - Q define two different axes of cross section. Z marks the zone which is depicted enlarged in Figure 6.
- **Figure 4**: This figure shows the L - L cross section of a device for performing the method of the invention along the central longitudinal axis. Reference number (10) cutting edge; (20) wall of the cylindrical or substantially cylindrical body; (30) grid; (40) shaft; (50) widening part; (60) handle.

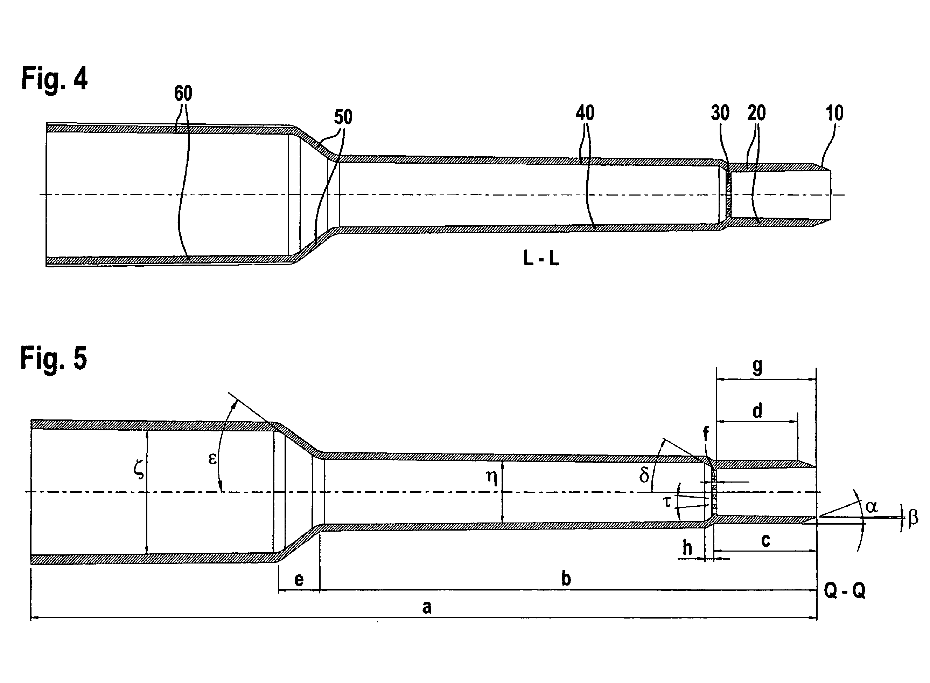

- **Figure 5**: This figure shows the Q - Q cross section of a device according to the invention along the central longitudinal axis. Angles are marked with greek letters, distances with roman letters. Angles and distances are given for a particularly preferred embodiment of the invention. Angles are measured in degrees (°) whereby an angle of 1° is understood as 1/360 of the circle circumference. The assumed measuring tolerance is ± 1°. (α) 20°; (β) 1°; (τ) 10°; (δ) 30°; (η) 1°; (ε) 36°; (ζ) 1°. Distances are given with an average measuring tolerance of ± 0,1 mm. (a) 95 mm; (b) 60 mm; (c) 12.4 mm; (d) 10 mm; (e) 5 mm; (f) 0.6 mm; (g) 12 mm;
- **Figure 6**: Enlarged section of the grip. Distances are given for a particularly preferred embodiment with an assumed measuring tolerance of [± 0,09 mm]; (g) 1.1 mm; (h) 0.3 mm.
- **Figure 7**: Dorsal view of the section of a bovine brainstem containing the obex region. Cranial: towards skull; caudal: towards spinal cord.

### Example 1

### Removing a tissue specimen from the obex of a slaughtered cow

In the slaughterhouse, the medulla oblongata or a part containing the obex region is withdrawn through the foramen magnum with the aid of a specially shaped spoon and stored in a special container. In the laboratory, the device according to the method of the invention is used to cut out an approximately cylindrical piece of tissue and to remove the latter from the section of brainstem. The cut-out piece of tissue is then removed from the device using tweezers and forwarded for analysis in order to test if appropriate for the presence of infectious prions.

When removing the piece of tissue, particular care is taken to ensure that the removed piece of tissue is free of blood clots.

### List of References

http://niah.naro.affrc.go.jp/disease/bse/retest_prionics.html
EP 1 293 167
US 2003/082797
US 2004/0116942
US 3,990,451

## Claims

1. Method for separating a tissue part (D) from the obex region of bovine brain material post mortem, whereby said brain material is from an animal slaughtered at an age of between 2 and 8 years, the method comprising the following steps:
(a) providing
(A) a smooth fixed support, and
(B) a device,
said device (B) being a hollow, tube-shaped entity comprising
(i) at the front end a circular blade (10) which can be pressed against the support, whereby the cutting edge of the circular blade tapers from the outside inwards, the circular blade continues as the wall of a cylindrical or substantially cylindrical body (20), at the end of which a grid (30) is arranged perpendicular to the cylinder axis, the distance between the blade edge and the grid is between 5 mm and 20 mm and the diameter of the circle formed by the blade is between 3 mm and 10 mm;
(ii) a cylindrical or substantially cylindrical shaft (40) with a length of between 20 mm and 100 mm, which is joined with the cylindrical body (20) on the other side of the grid (30);
(iii) at the rear end a cylindrical or substantially cylindrical handle (60) which is joined with the shaft by a widening part (50) with the surface of a truncated cone whose ends are circular, the end of the smaller circle diameter starting flush on the rear end of the shaft, and the end with the larger circle diameter starting flush on the handle;
(b) preparing a section of brainstem containing the obex region, said section of brainstem being placed with the ventral face on the smooth, fixed support (A); followed by
(c) placing the blade of the device (B) vertically on a predetermined site of the obex region; followed by
(d) pressing the blade in the vertical direction into the brain material while executing rotation movements, until the blade has made complete contact with the support; followed by
(e) pressing the device (B) against the support (A) and lifting the section of brainstem, so that tough or fibrous tissue components are separated from the tissue core surrounded by the cutting device;
(f) withdrawing the device (B) from the section of brainstem and removing the tissue part (D) contained in it.

2. Method according to claim 1, **characterized in that** in the device (B) the inner wall of the cylindrical body (20) forms the surface of a truncated cone whose ends are circular, the end of the smaller circle diameter facing the grid (30), and the end with the larger circle diameter being the circle formed by the blade, whereby the diameters of the smaller circle and the larger circle are selected such that they result in a truncated cone with an opening angle of between 0.1° and 5°.

3. Method according to any of the claims 1 and 2, **characterized in that** in the device (B) the cutting edge of the circular blade (10) tapers from the outside inwards at an angle of between 10° and 30°.

4. Method according to any of the claims 1 to 3, **characterized in that** in the device (B) the distance between blade edge (10) and grid (30) is between 5 mm and 20 mm and the diameter of the circle formed by the blade is between 3 mm and 10 mm.

5. Method according to any of the claims 1 to 4, **characterized in that** in the device (B) the grid (30) consists of between 15 and 30 elements with openings.

6. Method according to claim 5, **characterized in that** in the device (B) the width of each grid bar is between 0.2 mm and 1 mm.

7. Method according to claim 5, **characterized in that** in the device (B) the width of each grid bar is 0.4 mm.

8. Method according to any of the claims 1 to 7, **characterized in that** in step (e) the section of the brainstem is lifted to the widening part (50) of the cutting device (B).

9. Method according to any of the claims 1 to 8, **characterized in that** tweezers (C) are used in step (e) for lifting the brainstem, and in step (f) for removing the tissue part (D) from the device (B).

## Patentansprüche

1. Verfahren zur Trennung eines Gewebeteils (D) von der Obexregion von Rinderhirnmaterial post mortem, wobei das Hirnmaterial von einem Tier stammt, das im Alter zwischen 2 und 8 Jahren geschlachtet wurde, wobei das Verfahren die folgenden Schritte umfasst:
(a) Bereitstellung
(A) eines glatten festen Trägers und
(B) einer Vorrichtung,
wobei die Vorrichtung (B) ein hohles röhrenförmiges Objekt ist, das Folgendes umfasst:
(i) eine kreisförmige Klinge (10) am Vorderende, die gegen den Träger gedrückt werden kann, wobei die Schneidkante der kreisförmigen Klinge sich von außen nach innen verjüngt, wobei die kreisförmige Klinge sich als Wand eines zylindrischen oder im Wesentlichen zylindrischen Körpers (20) fortsetzt, an dessen Ende ein Gitter (30) lotrecht zur Zylinderachse angeordnet ist, wobei der Abstand zwischen der Klingenkante und dem Gitter zwischen 5 mm und 20 mm und der Durchmesser des von der Klinge geformten Kreises zwischen 3 mm und 10 mm beträgt;
(ii) einen zylindrischen oder im Wesentlichen zylindrischen Schaft (40) mit einer Länge zwischen 20 mm und 100 mm, der auf der anderen Seite des Gitters (30) mit dem zylindrischen Körper (20) verbunden ist;
(iii)einen zylindrischen oder im Wesentlichen zylindrischen Handgriff (60) am hinteren Ende, der mit dem Schaft über ein Aufweitungsteil (50) mit der Fläche eines Kegelstumpfes mit kreisförmigen Enden verbunden ist, wobei das Ende des kleineren Kreisdurchmessers bündig am hinteren Ende des Schafts beginnt und wobei das Ende mit dem größeren Kreisdurchmesser bündig am Handgriff beginnt;
(b) Präparieren eines Hirnstammabschnitts, der die Obexregion enthält, wobei der Hirnstammabschnitt mit der ventralen Seite auf den glatten fixierten Träger (A) gelegt wird; und anschließend
(c) vertikales Platzieren der Klinge der Vorrichtung (B) auf einer vorab festgelegten Stelle der Obexregion, wobei die Stelle mit (1) angegeben ist; und anschließend
(d) Drücken der Klinge in vertikaler Richtung in das Hirnmaterial unter Ausführen von Drehbewegungen, bis die Klinge vollständig mit dem Träger in Berührung ist; und anschließend
(e) Drücken der Vorrichtung (B) gegen den Träger (A) und Anheben des Hirnstammabschnitts, so dass zähe oder faserige Gewebebestandteile von dem Gewebekern, der von der Schneidvorrichtung umgeben ist, getrennt werden;
(f) Zurückziehen der Vorrichtung (B) vom Hirnstammabschnitt und Entfernen des darin enthaltenen Gewebeteils (D).

2. Verfahren nach Anspruch 1, **dadurch gekennzeichnet, dass** in der Vorrichtung (B) die Innenwand des zylindrischen Körpers (20) die Fläche eines Kegelstumpfes bildet, dessen Enden kreisförmige sind, wobei das Ende des kleineren Kreisdurchmessers zum Gitter (30) weist und das Ende mit dem größeren Kreisdurchmesser der Kreis ist, der von der Klinge geformt wird, wobei die Durchmesser des kleineren Kreises und des größeren Kreises so gewählt sind, dass sie einen Kegelstumpf mit einem Öffnungswinkel zwischen 0,1° und 5° ergeben.

3. Verfahren nach einem der Ansprüche 1 und 2, **dadurch gekennzeichnet, dass** in der Vorrichtung (B) die Schneidkante der kreisförmigen Klinge (10) sich von außen nach innen in einem Winkel zwischen 10° und 30° verjüngt.

4. Verfahren nach einem der Ansprüche 1 bis 3, **dadurch gekennzeichnet, dass** in der Vorrichtung (B) der Abstand zwischen der Klingenkante (10) und dem Gitter (30) zwischen 5 mm und 20 mm beträgt und der Durchmesser des von der Klinge geformten Kreises zwischen 3 mm und 10 mm ist.

5. Verfahren nach einem der Ansprüche 1 bis 4, **dadurch gekennzeichnet, dass** in der Vorrichtung (B) das Gitter (30) aus 15 bis 30 Elementen mit Öffnungen besteht.

6. Verfahren nach Anspruch 5, **dadurch gekennzeichnet, dass** in der Vorrichtung (B) die Breite jedes Gitterbalkens zwischen 0,2 mm und 1 mm beträgt.

7. Verfahren nach Anspruch 5, **dadurch gekennzeichnet, dass** in der Vorrichtung (B) die Breite jedes Gitterbalkens 0,4 mm beträgt.

8. Verfahren nach einem der Ansprüche 1 bis 7, **dadurch gekennzeichnet, dass** in Schritt (e) der Hirnstammabschnitt zum Aufweitungsteil (50) der Schneidvorrichtung (B) angehoben wird.

9. Verfahren nach einem der Ansprüche 1 bis 8, **dadurch gekennzeichnet, dass** eine Pinzette (C) in Schritt (e) zum Anheben des Hirnstamms und in Schritt (f) zum Entfernen des Gewebeteils (D) von der Vorrichtung (B) verwendet wird.

## Revendications

1. Procédé de séparation d'une partie de tissu (D) de la région de l'obex d'une matière cérébrale bovine *post mortem*, dans lequel ladite matière cérébrale provient d'un animal abattu à un âge d'entre 2 et 8 ans, le procédé comprenant les étapes suivantes :
(a) prévision
(A) d'un support fixe lisse, et
(B) un dispositif,
ledit dispositif (B) étant une entité creuse en forme de tube comprenant
(i) au niveau de l'extrémité avant une lame circulaire (10) qui peut être pressée contre le support, dans laquelle le bord coupant de la lame circulaire diminue progressivement de l'extérieur vers l'intérieur, la lame circulaire se poursuit comme la paroi d'un corps cylindrique ou substantiellement cylindrique (20), à l'extrémité duquel une grille (30) est agencée perpendiculairement à l'axe de cylindre, la distance entre le bord de la lame et la grille est entre 5 mm et 20 mm et le diamètre du cercle formé par la lame est entre 3 mm et 10 mm ;
(ii) un arbre cylindrique ou substantiellement cylindrique (40) avec une longueur d'entre 20 mm et 100 mm, qui est joint avec le corps cylindrique (20) sur l'autre côté de la grille (30) ;
(iii) à l'extrémité arrière une poignée cylindrique ou substantiellement cylindrique (60) qui est jointe avec l'arbre par une partie s'élargissant (50) avec la surface d'un cône tronqué dont les extrémités sont circulaires, l'extrémité du diamètre de cercle plus petit partant de niveau sur l'extrémité arrière de l'arbre, et l'extrémité avec le diamètre de cercle plus grand partant de niveau sur la poignée ;
(b) préparation d'une coupe de tronc cérébral contenant la région de l'obex, ladite coupe de tronc cérébral étant placée avec la face ventrale sur le support fixe lisse (A) ; suivie par
(c) le placement de la lame du dispositif (B) verticalement sur un site prédéterminé de la région de l'obex, ladite région étant indiquée par (1); suivi par
(d) la pression de la lame dans le sens vertical dans la matière cérébrale tout en exécutant des mouvements de rotation, jusqu'à ce que la lame soit en contact complet avec le support ; suivie par
(e) la pression du dispositif (B) contre le support (A) et le soulèvement de la coupe de tronc cérébral, de sorte que des composants de tissus durs ou fibreux sont séparés du coeur de tissu entouré par le dispositif coupant ;
(f) retrait du dispositif (B) de la coupe de tronc cérébral et prélèvement de la partie de tissu (D) contenue dans celui-ci.

2. Procédé selon la revendication 1, **caractérisé en ce que** dans le dispositif (B), la paroi interne du corps cylindrique (20) forme la surface d'un cône tronqué dont les extrémités sont circulaires, l'extrémité du diamètre de cercle plus petit faisant face à la grille (30), et l'extrémité avec le diamètre de cercle plus grand étant le cercle formé par la lame, dans lequel les diamètres du cercle plus petit et du cercle plus grand sont choisis de telle façon qu'ils résultent en un cône tronqué avec un angle d'ouverture d'entre 0,1° et 5°.

3. Procédé selon l'une quelconque des revendications 1 et 2, **caractérisé en ce que** dans le dispositif (B), le bord coupant de la lame circulaire (10) diminue progressivement de l'extérieur vers l'intérieur selon un angle d'entre 10° et 30°.

4. Procédé selon l'une quelconque des revendications 1 à 3, **caractérisé en ce que** dans le dispositif (B), la distance entre le bord de la lame (10) et la grille (30) est entre 5 mm et 20 mm et le diamètre du cercle formé par la lame est entre 3 mm et 10 mm.

5. Procédé selon l'une quelconque des revendications 1 à 4, **caractérisé en ce que** dans le dispositif (B), la grille (30) consiste en entre 15 et 30 éléments avec des ouvertures.

6. Procédé selon la revendication 5, **caractérisé en ce que** dans le dispositif (B), la largeur de chaque barre de grille est entre 0,2 mm et 1 mm.

7. Procédé selon la revendication 5, **caractérisé en ce que** dans le dispositif (B), la largeur de chaque barre de grille est 0,4 mm.

8. Procédé selon l'une quelconque des revendications 1 à 7, **caractérisé en ce qu'**à l'étape (e), la coupe du tronc cérébral est soulevée jusqu'à la partie s'élargissant (50) du dispositif coupant (B).

9. Procédé selon l'une quelconque des revendications 1 à 8, **caractérisé en ce que** des brucelles (C) sont utilisées à l'étape (e) pour soulever le tronc cérébral, et à l'étape (f) pour enlever la partie de tissu (D) du dispositif (B).
